# EUROPEAN PATENT SPECIFICATION

(11) **EP 1 268 056 B1**
(45) Date of publication and mention of the grant of the patent: **18.08.2004**
(21) Application number: 01933809.4
(22) Date of filing: 06.04.2001
(51) Int. Cl.: B01J 23/74, B01J 23/76, B01J 23/84, B01J 23/847, C07C 1/04

(54) **A PROCESS FOR PRODUCING HYDROCARBONS, AND A CATALYST SUITABLE FOR USE IN THE PROCESS**
VERFAHREN ZUR HERSTELLUNG VON KOHLENWASSERSTOFFEN UND FÜR DIESES VERFAHREN GEEIGNETER KATALYSATOR
PROCEDE DE PRODUCTION D'HYDROCARBURES ET CATALYSEUR APPROPRIE A CE PROCEDE

(30) Priority: 07.04.2000 EP 00302950
(43) Date of publication of application: 02.01.2003
(73) Proprietor: SHELL INTERNATIONALE RESEARCH MAATSCHAPPIJ B.V., 2596 HR Den Haag (NL)
(72) Inventor: VAN DEN BRINK, Peter, John, NL-1031 CM Amsterdam (NL); GEERLINGS, Jacobus, Johannes, Cornelis, NL-1031 CM Amsterdam (NL); HUISMAN, Hans, Michiel, NL-1031 CM Amsterdam (NL)
(86) International application number: PCT/EP2001/004072
(87) International publication number: WO 2001/076736

(56) References cited:
- GB-A- 579 427
- GB-A- 767 712
- GB-A- 873 449
- US-A- 2 254 806
- US-A- 2 553 433
- US-A- 3 660 514
- US-A- 3 793 225
- US-A- 3 810 953
- US-A- 4 654 458
- US-A- 5 080 872

## Description

The present invention relates to a process for producing hydrocarbons from synthesis gas and to a catalyst which is suitable for use in the process.

The catalytic preparation of hydrocarbons from synthesis gas, i.e. a mixture of carbon monoxide and hydrogen, is well known in the art and is commonly referred to as Fischer-Tropsch synthesis.

Supported catalysts suitable for use in a Fischer-Tropsch synthesis process typically contain a catalytically active metal of Group VIII of the Periodic Table of the Elements (Handbook of Chemistry and Physics, 68th edition, CRC Press, 1987-1988). In particular, iron, nickel, cobalt and ruthenium are well known catalytically active metals for such catalysts. Reference may be made to EP-A-398420, EP-A-178008, EP-A-167215, EP-A-168894, EP-A-363537, EP-A-498976 and EP-A-71770.

There is a continuous interest in finding catalysts which provide an improved selectivity in the conversion of carbon monoxide into valuable hydrocarbons, in particular hydrocarbons containing 5 or more carbon atoms ("C₅+ hydrocarbons" hereinafter), and which minimise the formation of carbon dioxide, which is a carbon containing by-product of low value.

It has now surprisingly been found that when in the Fischer-Tropsch synthesis a catalyst is used which contains inorganic phosphate more hydrocarbons, in particular more C₅+ hydrocarbons, and less carbon dioxide are produced. Further, there is less decline of the catalyst activity so that the catalysts may be used for a more prolonged period of time without the need for replacement or re-activation.

Accordingly, the present invention provides a process for producing C₅+ hydrocarbons, which process comprises contacting a mixture of carbon monoxide and hydrogen at elevated temperature and pressure with a catalyst comprising cobalt and manganese the catalyst being supported on a carrier, wherein the cobalt is present at least partly in metallic form and which catalyst further comprises inorganic phosphate in a quantity of at least 0.05 %w, calculated as elemental phosphorus relative to the weight of the catalyst.

A further metal may be present in order to improve the activity of the catalyst or the selectivity of the conversion of synthesis gas into hydrocarbons. Suitable further metals may be selected from vanadium, zirconium, rhenium and ruthenium. A preferred further metal is vanadium.

In a second aspect the present invention also provides in which the support is titania.

The amount of cobalt metal present in the catalyst may vary widely. Typically, the catalyst comprises from 1 to 50 %w cobalt metal, based on the weight of the metal relative to the weight of catalyst, preferably 3 to 40 %w, more preferably 5 to 30 %w on the same basis.

The amount of manganese or the further metal, if present, is typically from 0.05 and 60 %w, more typically from 0.1 to 25 %w, based on the weight of the metal relative to the weight of the catalyst.

The atomic ratio of the cobalt metal to the manganese or further metal, as present in the catalyst, is typically at least 5:1 and it is typically at most 200:1.

The inorganic phosphate may be present in the catalyst in any amount of at least 0.05 %w, calculated as elemental phosphorus, relative to the weight of the catalyst. Typically the inorganic phosphate will be present in an amount of less than 5 %w and a preferred amount is in the range of from 0.1 to 2.5 %w, for example 0.5 %w, on the same basis.

Generally a porous inorganic carrier may be found adequate for use in the present invention. In a preferred embodiment, the carrier is a refractory oxide carrier. Examples of suitable refractory oxide carriers include alumina, silica, titania, zirconia or mixtures thereof, such as silica-alumina or physical mixtures such as silica and titania. Preferably, the carrier comprises titania, alumina, zirconia or mixtures thereof, in particular titania or alumina.

The carrier comprising titania, alumina, zirconia or mixtures thereof, may further comprise up to 50 %w of another refractory oxide, typically silica, based on the total weight of the carrier. More preferably, the additional refractory oxide, if present, comprises up to 20 %w, even more preferably up to 10 %w, on the same basis.

The carrier most preferably comprises titania, in particular titania which has been prepared in the absence of sulphur-containing compounds. An example of such preparation method involves flame hydrolysis of titanium tetrachloride. It will be appreciated that the titania powder derived from such preparation method may not be of the desired size and shape. Thus, usually a shaping step is applied to prepare the carrier. Shaping techniques are well known to the skilled person and include palletising, extrusion, spray-drying and hot oil dropping methods.

The catalyst may suitably be prepared by methods known to the skilled person, such as by precipitating the catalytically active components or precursors onto the carrier; spray-coating, kneading, mulling and/or impregnating the catalytically active components or precursors onto the carrier; and/or extruding one or more catalytically active components or precursors together with carrier material to prepare catalyst extrudates. The term "catalytically active components" includes the cobalt metal, any further metal and the inorganic phosphate, as present in the catalyst. It is not excluded that, in addition to the catalytically active components and the carrier, the catalyst comprises further components.

It will be appreciated that the method of preparation may vary, depending e.g. on the desired size of the catalyst particles. It belongs to the skill of the skilled person to select the most suitable method for a given set of circumstances and requirements.

A preferred method of preparing the catalyst is by preparing a mixture of the carrier, the catalytically active components and/or precursors and a liquid, for example by kneading or mulling, shaping and drying the mixture thus obtained and subsequently calcining, such as disclosed in WO 99/34917.

A suitable liquid is water, ammonia, alcohols such as methanol and ethanol, ketones such as acetone, aldehydes such as propanal, and aromatic solvents such as toluene. Water is a preferred liquid.

The cobalt metal and the further metal may be employed in the form of the metal itself, or as an oxide, a hydroxide, or a salt such as a nitrate or an acetate, or mixtures thereof.

Suitable precursors of the phosphate as present in the catalyst are, for example, phosphorus compounds comprising an acid of phosphorus, such as metaphosphoric acid, pyrophosphoric acid, phosphorous acid, but preferably orthophosposphoric acid (H₃PO₄), or precursors of an acid of phosphorus, that is, a phosphorus containing compound capable of forming a compound containing at least one acidic hydrogen atom under circumstances where water is present or under such circumstances as applied in the preparation of the catalyst and/or in the activation of the catalyst, as set out hereinafter. A precursor of an acid of phosphorus is for example phosphorus pentoxide, phosphorus pentachloride or ammonium phosphates.

Calcination is typically carried out at a temperature between 200 and 900 °C, preferably, between 250 and 600 °C. The duration of the calcination treatment is typically from 0.5 to 24 hours, preferably from 1 to 4 hours. Suitably, the calcination treatment is carried out in an oxygen-containing atmosphere, preferably air. It will be appreciated that the average temperature during the calcination treatment will normally be higher than the average temperature during the drying treatment.

The catalyst is typically used to catalyse a process for the preparation of hydrocarbons from synthesis gas. Typically, when in use in that process, at least part of the cobalt is present in its metallic state. Preferably between 50 and 100 %w of the cobalt is present in its metallic state, especially between 60 and 98 %w.

Therefore, it is normally advantageous to activate the catalyst prior to use by a reduction treatment, in the presence of hydrogen at elevated temperature. Typically, the reduction treatment involves treating the catalyst at a temperature in the range from 100 to 450 °C, at elevated pressure, typically from 1 to 200 bar abs, frequently for 1 to 200 hours. Pure hydrogen may be used in the reduction treatment, but it is usually preferred to apply a mixture of hydrogen and an inert gas, like nitrogen. The relative amount of hydrogen present in the mixture may be in the range of from 0.1 to 100 %v.

According to a preferred embodiment, the catalyst is brought to the desired temperature and pressure level in a nitrogen gas atmosphere. Subsequently, the catalyst is contacted with a gas mixture containing only a small amount of hydrogen gas, the rest being nitrogen gas. During the reduction treatment, the relative amount of hydrogen gas in the gas mixture is gradually increased up to 50 %v or even 100 %v.

It may be preferred to activate the catalyst in-situ, that is inside the reactor for the preparation of hydrocarbons from synthesis gas. WO 97/17137 describes an in-situ catalyst activation process which comprises contacting the catalyst in the presence of hydrocarbon liquid with a hydrogen-containing gas at a hydrogen partial pressure of at least 15 bar abs., preferably at least 20 bar abs., more preferably at least 30 bar abs. Typically, in this process the hydrogen partial pressure is at most 200 bar abs.

The process for the preparation of hydrocarbons from synthesis gas is typically carried out at a temperature in the range of from 125 to 350 °C, preferably from 175 to 275 °C. The pressure is typically in the range of from 5 to 150 bar abs., preferably from 5 to 80 bar abs., in particular from 5 to 60 bar abs.

Hydrogen and carbon monoxide are typically fed to the process at a molar ratio in the range from 1 to 2.5. Low hydrogen to carbon monoxide molar ratios will increase the C₅+ selectivity, i.e. the selectivity of the formation of C₅+ hydrocarbons. The C₅+ selectivity of the process is preferably at least 90 %w based on the total amount of hydrocarbons produced, preferably 95%.

However, in the embodiment of the invention in which the metal is cobalt and the further metal is manganese and optionally vanadium, which are present in an atomic ratio of cobalt/(manganese + vanadium) of at least 12:1, the C₅+ selectivity of the catalyst is remarkably high, even when using synthesis gas having a high hydrogen to carbon monoxide atomic ratio. In this embodiment the hydrogen to carbon monoxide molar ratio is in the range from 1.5 to 2.5.

The gas hourly space velocity ("GHSV" hereinafter) may vary within wide ranges and is typically in the range from 400 to 10000 Nl/l/h, for example from 400 to 4000 Nl/l/h.

The term "GHSV" is well known in the art, and relates to the gas per hour space velocity, i.e. the volume of synthesis gas in Nl (i.e. at the standard temperature of 0 °C and the standard pressure of 1 bar (100,000 Pa)) which is contacted in one hour with one litre of catalyst particles, i.e. excluding interparticular void spaces. In the case of a fixed bed catalyst, the GHSV is usually expressed as per litre of catalyst bed, i.e. including interparticular void space. In that case a GHSV of 1600 Nl/l/h on catalyst particles corresponds to about 1000 Nl/l/h on catalyst bed.

The process for the preparation of hydrocarbons may be conducted using a variety of reactor types and reaction regimes, for example a fixed bed regime, a slurry phase regime or an ebullating bed regime. It will be appreciated that the size of the catalyst particles may vary depending on the reaction regime they are intended for. It belongs to the skill of the skilled person to select the most appropriate catalyst particle size for a given reaction regime.

Further, it will be understood that the skilled person is capable to select the most appropriate conditions for a specific reactor configuration, the reaction regime and a work-up scheme. For example, the preferred gas hourly space velocity may depend upon the type of reaction regime that is being applied. Thus, if it is desired to operate the hydrocarbon synthesis process with a fixed bed regime, preferably the gas hourly space velocity is chosen in the range from 500 to 2500 Nl/l/h. If it is desired to operate the hydrocarbon synthesis process with a slurry phase regime, preferably the gas hourly space velocity is chosen in the range from 1500 to 7500 Nl/l/h.

The invention will now be illustrated further by means of the following Examples.

### Example I

A mixture was prepared containing 112.5 g commercially available titania powder (P25 ex. Degussa), 49.5 g commercially available CO(OH)₂ powder, 8.2 g Mn(Ac)₂.4H₂O ("Ac" means acetate), 2.2 g phosphoric acid and 45 g water. The mixture was kneaded for 30 minutes. The mixture was shaped by means of an extruder. The extrudate were dried for 2 hours at 120 °C and calcined for 2 hours at 500 °C. The catalyst contained 22 %w CO, 1.2 %w Mn and 0.7 %w P.

The catalyst was tested in a process for the preparation of hydrocarbons. A micro-flow reactor containing 10 ml of the catalyst in the form of a fixed bed of catalyst particles was heated to a temperature of 260 °C, and pressurised with a continuous flow of nitrogen gas to a pressure of 2 bar abs. The catalyst was reduced in-situ for 24 hours with a mixture of nitrogen and hydrogen gas. During reduction the relative amount of hydrogen in the mixture was gradually increased from 0% to 100%. The water concentration in the off-gas was kept below 3000 ppmv.

Following reduction the pressure was increased to 31 bar abs. The preparation of hydrocarbons was carried out with a mixture of hydrogen and carbon monoxide at a H₂/CO ratio of 1.1:1. The GHSV amounted to about 930 Nl/l/h. The reaction temperature is expressed as the weighted average bed temperature was 205 °C. The space time yield, expressed as grams hydrocarbon product per litre catalyst particles (including the voids between the particles) per hour, was 100 g/l/h. The selectivity of CO₂, expressed in %mole CO₂ obtained relative to the number of moles CO converted; the selectivity of the hydrocarbons, expressed as %mole of carbon present in the hydrocarbons obtained relative to the number of moles CO converted; and the selectivity to hydrocarbons containing 5 or more carbon atoms (C₅+ selectivity), expressed as a weight percentage of the total hydrocarbon product, were determined after 30 hours of operation. The results are set out in Table I.

### Example II (comparative)

Example I was substantially repeated but with the difference that no phosphoric acid was present in the mixture.

**TABLE I**

| Example | I | II*) |
|---|---|---|
| Selectivity CO₂ (%mole) | 0.9 | 1.3 |
| Selectivity hydrocarbons (%mole) | 99.1 | 98.7 |
| C₅+ selectivity (%w) | 94 | 92 |

| | | |
|---|---|---|
| *): Comparative | | |

It will be appreciated that the catalyst of Example I, i.e. according to the invention, is much better than the catalyst of Example II, i.e. the comparative catalyst.

## Claims

1. A process for producing C5+ hydrocarbons, which process comprises contacting a mixture of carbon monoxide and hydrogen at elevated temperature and pressure with a catalyst comprising cobalt and manganese, the catalyst being supported on a carrier, wherein at least part of the cobalt is present in the metallic form and which catalyst further comprises inorganic phosphate in a quantity of at least 0.05 wt%, calculated as elemental phosphorous relative to the weight of the catalyst.

2. A process as claimed in claim 1, **characterised in that** a further metal is present, which further metal is selected from vanadium, zirconium, rhenium and ruthenium.

3. A process as claimed in claims 1 and 2, **characterised in that** inorganic phosphate is present in a quantity in the range of from 0.1 to 2.5 wt%, calculated as elemental phosphorus relative to the weight of the catalyst.

4. A process as claimed in any of claims 1-3, **characterised in that** the carrier comprises titania or alumina.

5. A Fischer-Tropsch catalyst which comprises, supported on a carrier which is titania, cobalt and manganese, which catalyst further comprises inorganic phosphate in a quantity of at least 0.05 wt%, calculated as elemental phosphorus relative to the weight of the catalyst, wherein at least part of the cobalt is present in the metallic form, the cobalt being present in an amount from 1 to 50 wt%, based on the weight of the metal relative to the weight of the catalyst.

6. A catalyst as claimed in claim 5, wherein the inorganic phosphate is present in a quantity in the range of 0.1 tot 2.5 wt%, calculated as elemental phosphorus relative to the weight of the catalyst.

7. A process for preparing a catalyst as claimed in claim 5, comprising the steps of preparing a mixture of the cobalt, manganese and the inorganic phosphate and/or precursors thereof, the carrier and a liquid, shaping and drying the prepared mixture and subsequently calcining, followed by a reduction treatment in the presence of hydrogen.

## Patentansprüche

1. Verfahren zur Herstellung von C₅⁺-Kohlenwasserstoffen, welches Verfahren ein Inkontaktbringen eines Gemisches aus Kohlenmonoxid und Wasserstoff bei erhöhter Temperatur und erhöhtem Druck mit einem Katalysator umfaßt, der Kobalt und Mangan, aufgebracht auf einen Träger, umfaßt, worin wenigstens ein Teil des Kobalts in metallischer Form zugegen ist und welcher Katalysator weiterhin anorganisches Phosphat in einer Menge von wenigstens 0,05 Gew.-%, berechnet als elementarer Phosphor und bezogen auf das Gewicht des Katalysators, umfaßt.

2. Verfahren nach Anspruch 1, **dadurch gekennzeichnet, daß** ein weiteres Metall zugegen ist, welches weitere Metall unter Vanadium, Zirkonium, Rhenium und Ruthenium ausgewählt ist.

3. Verfahren nach den Ansprüchen 1 und 2, **dadurch gekennzeichnet, daß** das anorganische Phosphat in einer Menge im Bereich von 0,1 bis 2,5 Gew.-%, berechnet als elementarer Phosphor und bezogen auf das Gewicht des Katalysators, zugegen ist.

4. Verfahren nach einem der Ansprüche 1 bis 3, **dadurch gekennzeichnet, daß** der Träger Titanoxid oder Aluminiumoxid umfaßt.

5. Ein Fischer-Tropsch-Katalysator, der Kobalt und Mangan umfaßt, aufgebracht auf einen Träger, der Titanoxid ist, welcher Träger weiterhin anorganisches Phosphat in einer Menge von wenigstens 0,05 Gew.-%, berechnet als elementarer Phosphor und bezogen auf das Gewicht des Katalysators, umfaßt, worin wenigstens ein Teil des Kobalts in metallischer Form zugegen ist, wobei das Kobalt in einer Menge von 1 bis 50 Gew.-%, bezogen auf das Gewicht des Metalles relativ zum Gewicht des Katalysators, zugegen ist.

6. Katalysator nach Anspruch 5, worin das anorganische Phosphat in einer Menge im Bereich von 0,1 bis 2,5 Gew.-% zugegen ist, berechnet als elementarer Phosphor und bezogen auf das Gewicht des Katalysators.

7. Verfahren zur Herstellung eines Katalysators nach Anspruch 5, das die Schritte des Ausbildens eines Gemisches aus Kobalt, Mangan und anorganischem Phosphat und/oder deren Vorläufern, aus dem Träger und aus einer Flüssigkeit, das Verformen und Trocknen des bereiteten Gemisches und anschließend ein Kalzinieren umfaßt, worauf eine Reduktionsbehandlung in Gegenwart von Wasserstoff folgt.

## Revendications

1. Procédé de production d'hydrocarbures en C₅+, lequel procédé comprend la mise en contact d'un mélange de monoxyde de carbone et d'hydrogène à température et pression élevées avec un catalyseur comprenant du cobalt et du manganèse, le catalyseur étant fixé sur un support, dans lequel au moins une partie du cobalt est présente sous la forme métallique, et lequel catalyseur comprend de plus du phosphate inorganique en une quantité d'au moins 0,05% en poids, calculé comme phosphore élémentaire par rapport au poids du catalyseur.

2. Procédé suivant la revendication 1, **caractérisé en ce qu'**un autre métal est présent, lequel autre métal est choisi parmi le vanadium, le zirconium, le rhénium et le ruthénium.

3. Procédé suivant l'une ou l'autre des revendications 1 et 2, **caractérisé en ce que** du phosphate inorganique est présent en une quantité allant de 0,1 à 2,5% en poids, calculé comme phosphore élémentaire par rapport au poids du catalyseur.

4. Procédé suivant l'une quelconque des revendications 1 à 3, **caractérisé en ce que** le support comprend de l'oxyde de titane ou de l'alumine.

5. Catalyseur de Fischer-Tropsch qui comprend, fixé sur un support qui est de l'oxyde de titane, du cobalt et du manganèse, lequel catalyseur comprend de plus du phosphate inorganique en une quantité d'au moins 0,05% en poids, calculé comme phosphore élémentaire par rapport au poids du catalyseur, dans lequel au moins une partie du cobalt est présente sous la forme métallique, le cobalt étant présent en une quantité de 1 à 50% en poids, sur la base du poids du métal par rapport au poids du catalyseur.

6. Catalyseur suivant la revendication 5, dans lequel le phosphate inorganique est présent en une quantité allant de 0,1 à 2,5% en poids, calculé comme phosphore élémentaire par rapport au poids du catalyseur.

7. Procédé de préparation d'un catalyseur suivant la revendication 5, comprenant les étapes de préparation d'un mélange du cobalt, du manganèse et du phosphate inorganique et/ou de leurs précurseurs, du support et d'un liquide, de façonnage et de séchage du mélange préparé et ensuite de calcination, suivis d'un traitement de réduction en présence d'hydrogène.
